# EUROPEAN PATENT SPECIFICATION

(11) **EP 3 246 065 B1**
(45) Date of publication and mention of the grant of the patent: **25.08.2021**
(21) Application number: 16305581.7
(22) Date of filing: 20.05.2016
(51) Int. Cl.: A61M 16/06

(54) **LIGHT PEDIATRIC RESPIRATORY MASK**
LEICHTE PÄDIATRISCHE ATEMMASKE
MASQUE RESPIRATOIRE PÉDIATRIQUE LÉGER

(43) Date of publication of application: 22.11.2017
(73) Proprietor: Air Liquide Medical Systems, 92160 Antony (FR)
(72) Inventor: ALBERICI, Luca, 25030 RONCADELLE (BS) (IT); MASSERDOTTI, Fulvio, 25075 BRESCIA (IT); SANDONI, Giuseppe, 25124 BRESCIA (IT)
(74) Representative: Air Liquide

(56) References cited:
- EP-A1- 2 818 194
- WO-A1-2007/045023
- WO-A1-2008/037031
- US-A1- 2010 192 954

## Description

The invention concerns a light and flexible pediatric respiratory nasal mask for use in the treatment of respiratory conditions or diseases affecting infants, children, toddlers, babies, newborns or the like, whatever their facial morphology.

Nasal masks are commonly used for delivering non-invasive positive pressure ventilation (NPPV) or for nasal continuous positive airway pressure (N-CPAP) therapy in disordered breathing conditions, such as obstructive sleep apnea (OSA), chronic obstructive pulmonary disease (COPD)...

Nasal masks deliver a flow of breathable gas for, or to assist in, patient respiration. Such a mask assembly typically comprises a rigid or semi-rigid hollow shell, usually made of polymer, defining a breathing chamber that receives at least a part of the patient's nose and further comprising a soft face-contacting cushion that comes into contact with the patient's face and conforms to the various facial contours of the patient face, which cushion is usually made of soft, resilient elastomeric material, such as soft silicone or similar, and a forehead support and a headgear for correctly positioning, maintaining and/or securing the mask on the head of a patient.

The forehead support is usually arranged on an expansion part of the mask, i.e. a portion of the mask body forming a holding arm that projects upwardly from the mask body and in the direction of the forehead of the user when the mask is positioned on the user's face. The hollow shell typically receives a gas supply line which delivers a respiratory gas, such as air under pressure, into the breathing chamber of the shell. An example of a nasal mask of this kind is given by EP-A-462701.

The mask assembly is secured to the wearer's head by straps or similar devices thereby forming a headgear that can be adjusted to pull the mask against the face with sufficient force to achieve a gas tight seal between the mask and the wearer's face as taught by documents EP-A-462701, EP-A-874667, EP-A-1972357 or WO-A-00/57942.

Such respiratory masks have been mainly developed for adults patients. This is why problems arise when respiratory masks have to be used in the pediatric field.

Indeed, respiratory masks that are designed for adults cannot be used for delivering gases to children, babies or the like as their facial morphologies are very different, which involves constraints for well positioning and maintaining the mask on the child or baby's face, leading to discomfort and to other issues for the patients.

Further, pediatric masks designed for newborns, babies or the like, that typically have a weight of between about 2.5 and 12 kg, are usually not adapted to older children or infants. Indeed, the facial morphologies of children or toddlers of one or several years compared to the ones of infants/babies /newborns of only one or several days, weeks or months are usually very dissimilar and evolve quickly while the baby/child is growing up. This becomes an important problem when the child/infant should receive a gaseous treatment delivered by a mask because the mask should well-fit with the facial morphology of the patient, whatever his/her age, so as to get an efficient gas delivery and not impairing the gaseous treatment delivered by the mask.

Furthermore, pediatric masks that have to be used for delivering gas to babies or the like, i.e. young infants weighting less than 12 kg, have to be very light for being wellaccepted by the babies and for not disturbing them in creating a discomfort while the gas is delivered. The masks should also be made in a material that is harmful for the babies or the like.

Such constraints explain why a lot of current masks are not well-adapted to pediatric usages.

Document WO 2008/037031 A1 proposes a respiratory nasal mask which is integrally moulded in one piece from an elastomeric material such as silicone rubber. Advantageously, the mask may be compressed into an approximate ball shape using a moderate level of hand/digital pressure, and may weigh less than 50 grams including the weight of the straps.

Document EP-A-2818194 proposes a pediatric nasal mask which is integrally moulded in one piece from a resilient soft material, such as silicone. The mask body has a generally triangular tridimensional shape and comprises an inlet orifice which is arranged at the center of the mask body.

Document EP-A-2114500 proposes a pediatric nasal mask including a flexible cushion with a tube connection portion and a rigid support structure adjacent the cushion and a headgear for stabilizing the cushion and preventing it from collapsing. This document does not take into account the very variable morphology of babies, newborns, children, infants, toddlers and the like.

In other words, today existing pediatric masks, especially nasal masks, are not satisfying and need to be improved to better account for the very variable morphology of newborns, babies, children and infants thereby ensuring efficient gas tightness (seal) and increased comfort and/or avoiding the possibility to have face deformations or injuries due to a not well-adapted mask structure.

Hence, the problem to be solved is to provide an improved pediatric mask architecture ensuring efficient gas tightness (seal) and/or increased comfort for baby, infant, a toddler or a child.

The solution of the present invention concerns a pediatric respiratory mask comprising:
- a mask body comprising an internal chamber and an inlet orifice in fluid communication with the internal chamber,
- a holding arm arranged on the mask body and projecting upwardly from said mask body,
- a cushion having an aperture adapted for receiving at least part of the patient's nose, when the patient wears the mask, and
- two lateral arms arranged on each side of the mask body and projecting laterally from said mask body,
and wherein the mask body, the holding arm, the cushion and the two lateral arms are integral and form a single mask structure made of a resilient soft material, wherein the mask is a nasal mask dimensioned for fitting to the nasal region of an infant, newborn, baby, toddler or child, and wherein:
- the single mask structure is molded in one piece made of said resilient soft material,
- the mask body has a triangular or trapezoidal general shape, and
- the inlet orifice is arranged at the center of the mask body,
characterized in that:
- the single mask structure has a weight of less than 30 g, and
- the mask body has a triangular or trapezoidal general shape having a first width (L1) of less than 60 mm and a first height (H1) of less than 60 mm.

The pediatric mask according to the present invention can further comprise one or more of the following additional features:
- the single mask structure has a weight of between 10 and 20 g.
- the single mask structure has a weight of between 12 and 19 g.
- one of the two lateral arms projects from the right side of the mask body, while the other of the two lateral arms projects from the left side of the mask body.
- the resilient soft material forming the single mask structure comprises silicone, or the like, preferably silicone.
- the single mask structure is entirely flexible.
- the mask body has a first width L1 of less than 50 mm.
- the mask body has a first width L1 of between 40 and 48 mm.
- the mask body has a first height H1 of less than 50 mm.
- the mask body has a first height H1 of between 30 and 45 mm.
- the mask body and the two lateral arms arranged on each side of the mask body have a second width L2 of less than 110 mm, preferably less than 100 mm.
- the second width L2 is of between 82 and 99 mm.
- the second height H2 (i.e. distance) measured between the plan containing the lower apex of the two lateral arms, and the plan containing the upper apex of the holding arm is of less than 120 mm, preferably less than 110 mm.
- the second height H2 is of between 80 and 105 mm, typically between 85 and 103 mm.
- the inlet orifice of the mask body has an inner diameter D of between 12 and 19 mm, preferably of between 14 and 17 mm.
- the mask body has a thickness T of less than 45 mm, preferably less than 40 mm, typically of between 25 and 35 mm, preferably between 30 and 35 mm.
- the cushion comprises at least one flexible membrane, said membrane being made of said resilient soft material and is integral with the cushion.
- an annular element is arranged in the inlet orifice of the mask body, preferably an annular element made of a rigid polymer, such as plastic.
- the holding arm projecting upwardly from said mask body comprises at least one slot at its terminal end, typically several slots, such as 3 slots, for fixing thereto straps of a headgear.
- at least one of the two opposite lateral arms projecting laterally from said mask body comprises a slot, preferably both lateral arms comprise a slot for fixing thereto straps of a headgear.
- the mask further comprises a headgear.
- the mask further comprises connecting elements for fixing the headgear to the mask body, such as one or several straps. The one or several straps of the headgear cooperate with one (or more) of the slots arranged at the terminal end of the holding arm and/or in the two lateral arms.
- the mask body has a generally triangular or trapezoidal tridimensional shape.
- the mask body has preferably a generally triangular.
- the holding arm projecting upwardly from the mask body and the two opposite lateral arms projecting laterally from said mask body are connected to the corners or angle regions of the triangular-shaped mask body.
- the two opposite lateral arms have a generally curved-shape, preferably the two lateral arms are curved toward the rear and bottom side of the mask body.
- the cushion comprises an aperture having a triangular form or other similar shapes that is configured for receiving the nose region of the pediatric patient and thus ensuring gas tightness and good comfort of use.

Further, the invention also concerns an assembly comprising a gas delivery device, such as a medical ventilator, and a pediatric respiratory mask according to the present invention. Preferably, the gas delivery device is connected to the respiratory mask by means of a gas line, such as a flexible hose, for conveying and delivering a respiratory gas to the mask, said gas being subsequently inhaled by a pediatric patient.

Generally speaking, a pediatric mask according to the present invention is considerably much lighter than the masks of the prior art, for instance it can be about 8 to 10 times lighter than a classical respiratory mask used for treating adults.

Further, thanks to its very specific dimensions/sizes and its particular design, a pediatric mask according to the present invention perfectly matches the morphologies of pediatric patients thereby improving the comfort of use and ensuring efficient gas tightness.

Preferred embodiments of a pediatric nasal mask according to the present invention are shown in the enclosed Figures, among which :
- Figures 1 and 2 represent front and lateral views of a first embodiment of a nasal mask according to the present invention that is designed for babies,
- Figures 3 and 4 represent front and rear views of the mask of Figures 1 and 2,
- Figures 5 and 6 represent front and lateral views of a second embodiment of a nasal mask according to the present invention that is designed for children, and
- Figures 7 and 8 represent front and rear views of the mask of Figures 5 and 6.

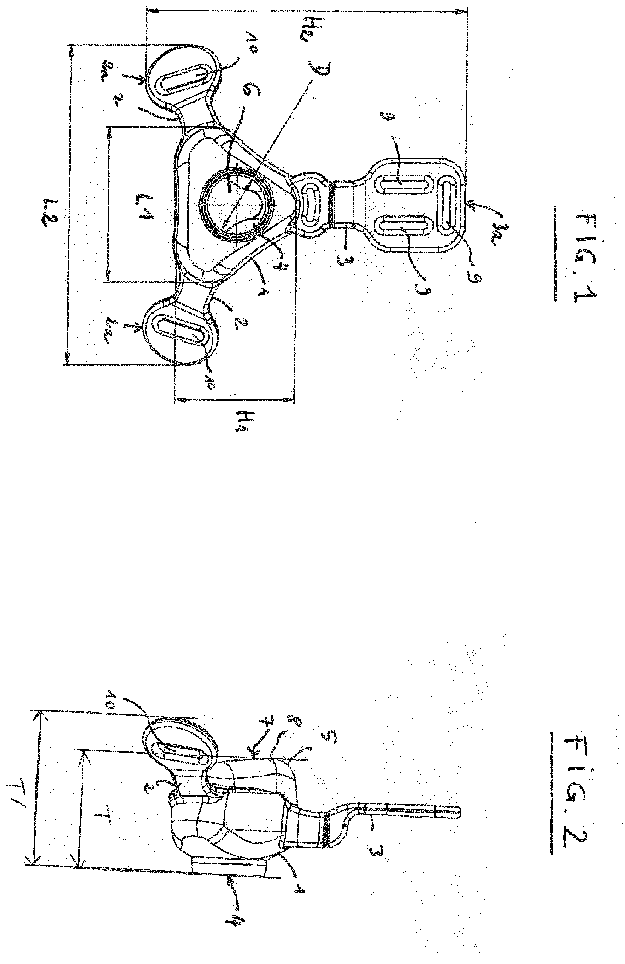

The present invention proposes a new type of respiratory nasal mask, especially designed and configured for being used in the pediatric field, as illustrated in Figures 1 to 8 that illustrate two embodiments of a mask body 1 according to the present invention that is configured to a pediatric usage. As the masks of Figures 1-8 have very similar structures and comprise common features, same references are used hereafter for designating identical parts or elements of those masks.

As shown in Fig. 1-8, a pediatric nasal mask according to the present invention comprises a hollow shell or mask body 1 defining an internal breathing chamber 6 or volume, wherein respiratory gas, such as air under pressure, is introduced via an inlet port 4 to which is connected a gas feeding line, such as a flexible hose, by means of a tubular connector or similar.

The gas inlet orifice 4 is arranged at the center of the front side of the mask body 1 and through its wall, thereby allowing gas under pressure to be introduced into the breathing chamber 6 as said inlet orifice 4 is in fluid communication with chamber 6.

Preferably, the inlet orifice 4 has an inner diameter D of between 12 and 19 mm, preferably of between 14 and 17 mm.

The mask body 1 has a generally triangular or trapezoidal tridimensional shape as visible in Figures 1-8 so as to better match the morphology of the nasal regions of the patient.

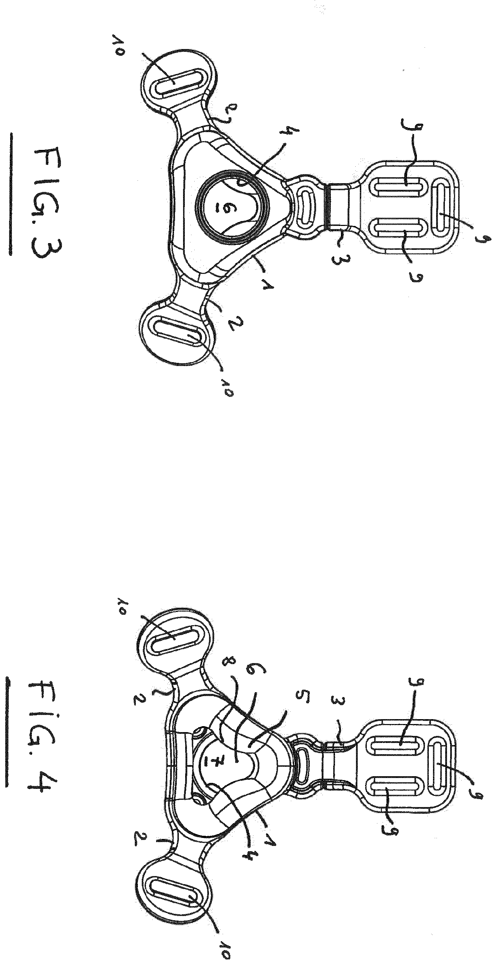

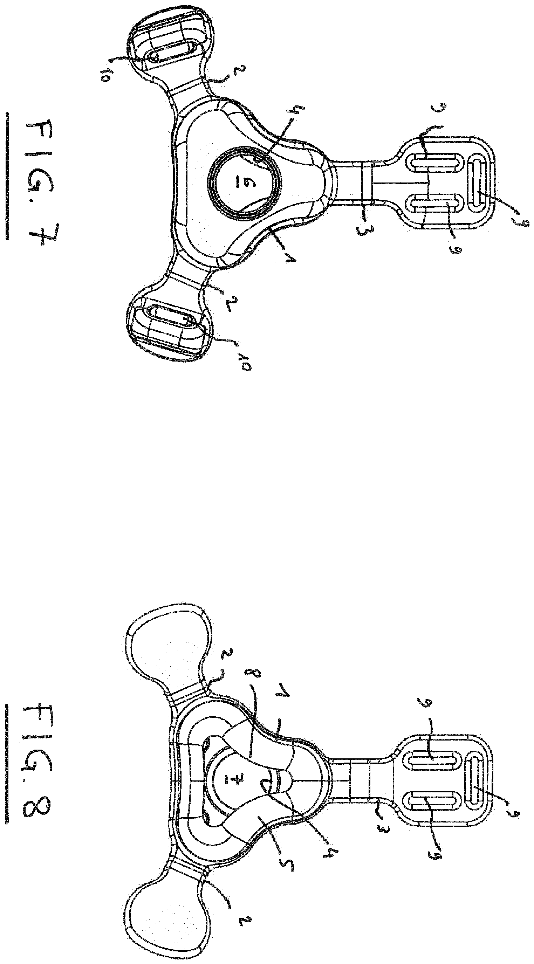

The mask body 1 receives at least a part of the patient's nose, when said patient introduces his/her nose into the internal volume of the breathing chamber 6 of the mask body 1, through an aperture 7 situated at the rear of the mask body 1 as shown in Figures 4 and 8, for breathing of the gas contained therein.

More precisely, the aperture 7 is arranged in a cushion 5 that is designed for receiving at least part of the patient's nose, when the patient wears the mask, thereby ensuring gas tightness. Additional details are given below.

Further, the mask body 1 also comprises, on the one hand, a holding arm 3 forming an upper or frontal support that is integral with the mask body 1 and projects upwardly from said mask body 1 and, one the other hand, two lateral arms or wings 2 that are also integrally arranged on the mask body 1 and that project laterally, i.e. on each opposite lateral sides (right and left sides) from said mask body 1. Those lateral arms 2 may also constitute right and left cheek supports, when the mask is worn by a patient. They are also used for connecting a headgear thereto, as explained below.

According to the present invention, in order to provide efficient gas tightness (seal) and/or increased comfort for a pediatric patient, i.e. when the patient is a newborn, baby, toddler, infant, child or the like, the mask of the present invention, as shown in Fig. 1-8, has been designed for being very light.

Thus, the weight of the mask body 1, the holding arm 3, the cushion 5 and the two lateral arms 2 that constitute the main elements of the mask, is less than 30 g, more preferably less than 20 g, i.e. the lighter the better.

In this goal, the mask has been conceived so that the mask body 1, the holding arm 3, the cushion 5 and the two lateral arms 2 are made of a single piece of a resilient soft material, such as preferably silicone.

They are molded in one piece so as to constitute a single mask piece or structure of resilient soft material, i.e. silicone, that is very light and can be worn easily by pediatric patients, including very young babies, such as newborns, while limiting the discomfort for the pediatric patients.

Said single mask structure of resilient soft material is entirely flexible and can hence be adapted to a great variety of facial morphologies, especially of various nasal regions of infant, child, baby, newborn or toddler patients.

Using a soft material also avoids the risks that some pediatric patients encounter facial deformations or be harmed due to an excessive rigidity of the mask body 1 or any other element of it.

The soft resilient cushion 5, that comes into contact with the patient's face, during use of the mask, and which comprises the central aperture 7 for receiving the patient's nose, preferably also comprises a soft flexible membrane 8 forming a kind of skirt delimits said central aperture 7. Said soft flexible membrane 8 is also molded in one piece with the rest of the cushion 5 so as to form part of the single piece of flexible material.

When present, said flexible membrane 8 should not negatively impact the total weight of the single mask structure than should be kept in the above limits. Of course, depending on the embodiment, the cushion 5 can comprise several superimposed membranes 8, such as two superimposed membranes. Using several membranes may improve the gas tightness. Nevertheless, in the embodiments of Fig. 1-8, a unique membrane 8 is provided as using several membranes is not necessary.

According to the present invention, the entire mask is light and flexible as formed of the same resilient material, namely silicone, i.e. the single mask structure including the mask body 1, the holding arm 3, the cushion 5, membrane 8, and the two lateral arms 2.

Further, as already mentioned, the mask body 1 has a triangular or trapezoidal general shape as illustrated in Fig. 1-8.

In the frame of the present invention, it is also very important to precisely dimension/size the mask body 1 as well as the two lateral arms 2 and the holding arm 3 that are integral with the mask body 1, so that the mask is well-adapted for a use in the pediatric field, i.e. with different facial morphologies of young patients, i.e. newborns, babies, toddlers, infants, children, or the like.

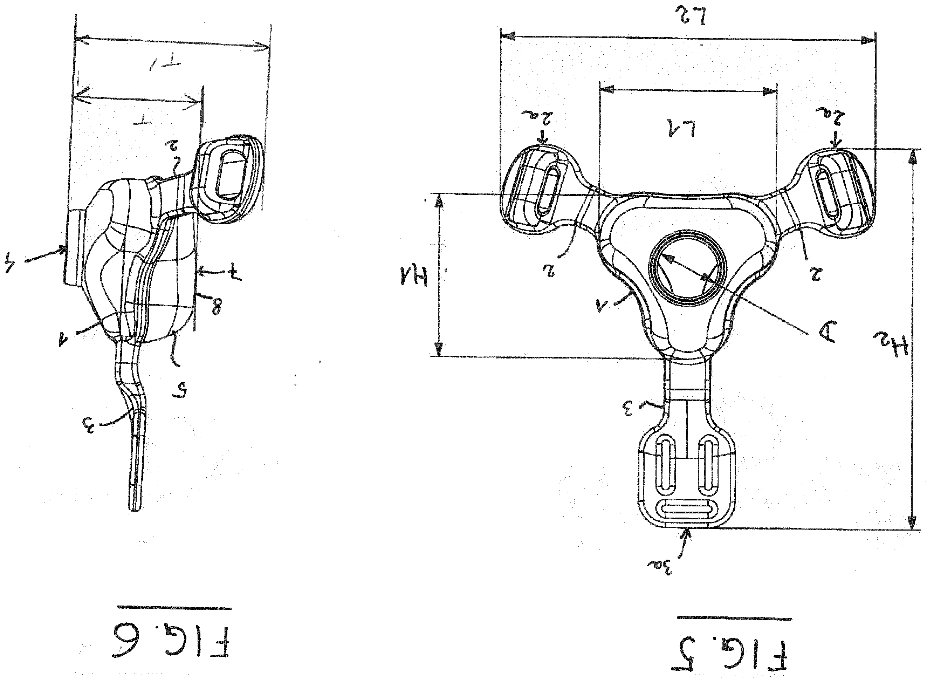

In this goal, as shown in Figures 1 and 5, the mask body 1 should have :
- a first width L1 of less than 60 mm, preferably less than 50 mm, typically of between 38 and 48 mm,
- a first height H1 of less than 60 mm, preferably less than 50 mm, typically of between 30 and 45 mm.
- a second width L2 corresponding to the width first H1 of the mask body 1 and the length of the two lateral arms 2 arranged on each side of the mask body 1 that should be chosen so as to be of less than 110 mm, preferably less than 100 mm, typically of between 80 and 99 mm.
- a second height H2 measured between the plan containing the lower apex 2a of the two lateral arms 2 and the plan containing the upper apex 3a of the holding arm 3 (cf. Fig. 1 and 5). The second height H2 is of less than 120 mm, preferably less than 110 mm, typically of between 90 and 105 mm.

The second width L2 and the second height H2 are preferably measured when the mask lays on a plane surface as shown in Fig. 1 and 5.

Furthermore, as the mask is to be used for treating pediatric patients, it should not be too thick. Hence, it is important to choose the thickness T of the mask body 1 and cushion 8 between 25 and 45 mm, typically between 30 and 40 mm, preferably between 30 and 35 mm. Further, the total thickness T' of the mask considering the mask body and the two lateral arms 2 is of less than 60 mm, preferably less than 55 mm, typically of about 40 to 50 mm. The thicknesses T and T' are measured as shown in Fig. 2 and 6.

The following Table gives examples of dimensions to be applied to two nasal masks for pediatric usages according the present invention.

**Table**

| Type of mask/patient (patient weight) | Nasal mask for babies (2,5 - 12 kg) | Nasal mask for children (> 12 kg) |
|---|---|---|
| Material of the one-piece mask structure | Molded silicone | Molded silicone |
| General shape of mask body | Triangular | Triangular |
| Shown in | Figure 1 | Figure 5 |
| Total weight of the one-piece mask structure (*) | 12,5 g | 18,6 g |
| L1 | 41,5 mm | 45,8 mm |
| H1 | 32,4 mm | 42,9 mm |
| L2 | 85,5 mm | 96,9 mm |
| H2 | 86,8 mm | 100,3 mm |
| D | 15,8 mm | 15,8 mm |
| T | 31,6 mm | 33,8 mm |
| T' | 41,6 mm | 50,0 mm |

| | | |
|---|---|---|
| (*) The one-piece mask structure considered for measuring the weight is the single mask structure shown in Fig. 1-8, without any additional elements, such as headgear, elbow connector... | | |

The respiratory mask according to the present invention is a nasal mask (Fig. 1-8). The cushion 5 and the membrane 8 comprise an upper nasal bridge region, a lower region and two lateral regions (i.e. left and right regions) connecting the nasal bridge and lower regions. When the mask is worn by the patient, the upper nasal bridge region is in contact with the nasal bridge of the patient, the lower region is in contact with the area between the nose and the upper lip of the patient, and the lateral opposite regions are in contact with the flange regions of the nose of the patient.

As shown in Fig. 1 and 5, the mask body 1 of the two embodiments according to the present invention, has a generally triangular tridimensional shape for better matching or corresponding to the general structure of a nose of a pediatric patient. The holding arm 3 projects upwardly from one la of the three corners (i.e. angle region) of the triangular structure of the mask body 1, whereas the two lateral arms 2 project laterally from the two other corners of said mask body 1.

Further, the holding arm 3 comprises one or several slots 9 or similar traversing orifices or holes of any shapes, preferably located at its terminal end, for receiving and fixing one or several straps of a headgear (not shown). Similarly, the two lateral arms 2 comprises one (or several) slot or any other connecting structure 10 for fixing thereto the straps of a headgear (not shown). Indeed, a headgear is generally connected to the holding arm 3 and to the two lateral arms 2 for maintaining the mask in a desired position on the head of the patient during its use and thus obtaining an efficient treatment of respiratory disorders.

Further, the flexible shell or mask body 1 is fluidly linked to a gas supply line, such as a flexible hose, by means of the tubular hollow connector, such as an elbow connector, which delivers a respiratory gas, such as air under pressure, into the breathing chamber 6 of the mask body 1. The gas supply line is fed with gas under pressure by a respiratory device or ventilator (not shown). The tubular hollow connector can be connected to the mask body 1 by means of an annular element, such a polymer (plastic) ring or similar, that can be arranged in the inlet orifice 4 of the mask body 1 that is located on the front side of the mask body.

A nasal respiratory mask of the present invention can be used in a method for treatment of a respiratory disorder or condition affecting pediatric patients, such as newborns, babies, infants, toddlers, children, or the like, in non-invasive positive pressure ventilation (NPPV) or any other respiratory disease affecting pediatric patients.

## Claims

1. Pediatric respiratory mask comprising:
- a mask body (1) comprising an internal chamber (6) and an inlet orifice (4) in fluid communication with the internal chamber (6),
- a holding arm (3) arranged on the mask body (1) and projecting upwardly from said mask body (1),
- a cushion (5) having an aperture (7) adapted for receiving at least part of the patient's nose, when the patient wears the mask, and
- two lateral arms (2) arranged on each side of the mask body (1) and projecting laterally from said mask body (1),
and wherein the mask body (1), the holding arm (3), the cushion (5) and the two lateral arms (2) are integral and form a single mask structure made of a resilient soft material, wherein the mask is a nasal mask dimensioned for fitting to the nasal region of an infant, newborn, baby, toddler or child, and wherein:
- the single mask structure is molded in one piece made of said resilient soft material,
- the mask body has a triangular or trapezoidal general shape, and
- the inlet orifice is arranged at the center of the mask body,
**characterized in that**:
- the single mask structure has a weight of less than 30 g, and
- the mask body has a triangular or trapezoidal general shape having a first width (L1) of less than 60 mm and a first height (H1) of less than 60 mm.

2. Mask according to Claim 1, **characterized in that** the mask body has a first width (L1) of less than 50 mm and a first height (H1) of less than 50 mm.

3. Mask according to Claim 1, **characterized in that** the single mask structure has a weight of between 10 and 20 g.

4. Mask according to any one of Claims 1 or 2, **characterized in that** the mask body has a first width (L1) of between 40 and 48 mm and a first height (H1) of between 30 and 45 mm.

5. Mask according to any one of the preceding Claims, **characterized in that** the resilient soft material forming the unique mask piece comprises silicone.

6. Mask according to Claim 1, **characterized in that** the single mask structure has a weight of between 12 and 19 g.

7. Mask according to any one of the preceding Claims, **characterized in that** the cushion (5) comprises at least one flexible membrane (8), said membrane (8) being made of said resilient soft material and integral with the cushion (5).

8. Mask according to Claim 1, **characterized in that** the mask body has a thickness (T) of less than 45 mm.

9. Mask according to Claim 1, **characterized in that** the mask body (1) has a generally triangular tridimensional shape.

10. Mask according to Claim 1 or 9, **characterized in that** the holding arm (3) projecting upwardly from the mask body (1) and the two opposite lateral arms (2) projecting laterally from said mask body (1) are connected to the corners or angle regions of the triangular-shaped mask body (1).

11. Mask according to Claim 1, **characterized in that** an annular element made of a rigid polymer is arranged in the inlet orifice (4) of the mask body (1).

12. Mask according to Claim 11, **characterized in that** the two opposite lateral arms (2) have a generally curved-shape, said two lateral arms (2) being curved toward the rear and bottom side of the mask body (1).

13. Mask according to Claim 7, **characterized in that** the cushion (5) and the membrane (8) comprise an upper nasal bridge region, a lower region and two lateral regions connecting the nasal bridge and lower regions, the upper nasal bridge region being in contact with the nasal bridge of the patient, the lower region being in contact with the area between the nose and the upper lip of the patient, and the lateral opposite regions being in contact with the flange regions of the nose of the patient, when the mask is worn by a patient,

14. Assembly comprising a gas delivery device and a respiratory mask according to any one of the preceding Claims.

15. Assembly according to Claim 14, **characterized in that** the gas delivery device is connected to the respiratory mask by means of a gas line, such as a flexible hose.

## Patentansprüche

1. Pädiatrische Atemmaske, umfassend:
- einen Maskenkörper (1), der eine Innenkammer (6) und eine mit der Innenkammer (6) in Fluidverbindung stehende Einlassöffnung (4) umfasst,
- einen Haltearm (3), der auf dem Maskenkörper (1) angeordnet ist und vom Maskenkörper (1) nach oben ragt,
- ein Polster (5) mit einer Öffnung (7), die dazu angepasst ist, mindestens einen Teil der Nase des Patienten aufzunehmen, wenn der Patient die Maske trägt, und
- zwei seitliche Arme (2), die auf jeder Seite des Maskenkörpers (1) angeordnet sind und seitlich vom Maskenkörper (1) vorragen,
und wobei der Maskenkörper (1), der Haltearm (3), das Polster (5) und die beiden seitlichen Arme (2) einstückig sind und eine einzelne, aus einem nachgiebigen weichen Material bestehende Maskenstruktur bilden,
wobei die Maske eine Nasenmaske ist, die solche Abmessungen aufweist, dass sie auf die Nasenregion eines Säuglings, Neugeborenen, Babys, Kleinkinds oder Kinds passt, und wobei:
- die einzelne Maskenstruktur in einem Stück aus dem nachgiebigen weichen Material geformt wird,
- der Maskenkörper eine dreieckige oder trapezförmige allgemeine Gestalt aufweist, und
- die Einlassöffnung in der Mitte des Maskenkörpers angeordnet ist,
**dadurch gekennzeichnet, dass**:
- die einzelne Maskenstruktur ein Gewicht von weniger als 30 g aufweist, und
- der Maskenkörper eine dreieckige oder trapezförmige allgemeine Gestalt mit einer ersten Breite (L1) von weniger als 60 mm und einer ersten Höhe (H1) von weniger als 60 mm aufweist.

2. Maske nach Anspruch 1, **dadurch gekennzeichnet, dass** der Maskenkörper eine erste Breite (L1) von weniger als 50 mm und eine erste Höhe (H1) von weniger als 50 mm aufweist.

3. Maske nach Anspruch 1, **dadurch gekennzeichnet, dass** die einzelne Maskenstruktur ein Gewicht zwischen 10 und 20 g aufweist.

4. Maske nach einem der Ansprüche 1 oder 2, **dadurch gekennzeichnet, dass** der Maskenkörper eine erste Breite (L1) zwischen 40 und 48 mm und eine erste Höhe (H1) zwischen 30 und 45 mm aufweist.

5. Maske nach einem der vorhergehenden Ansprüche, **dadurch gekennzeichnet, dass** das nachgiebige weiche Material, aus dem das einzige Maskenstück geformt ist, Silikon umfasst.

6. Maske nach Anspruch 1, **dadurch gekennzeichnet, dass** die einzelne Maskenstruktur ein Gewicht zwischen 12 und 19 g aufweist.

7. Maske nach einem der vorhergehenden Ansprüche, **dadurch gekennzeichnet, dass** das Polster (5) mindestens eine flexible Membran (8) umfasst, wobei die Membran (8) aus dem nachgiebigen weichen Material besteht und mit dem Polster (5) einstückig ist.

8. Maske nach Anspruch 1, **dadurch gekennzeichnet, dass** der Maskenkörper eine Dicke (T) von weniger als 45 mm aufweist.

9. Maske nach Anspruch 1, **dadurch gekennzeichnet, dass** der Maskenkörper (1) eine allgemein dreieckige dreidimensionale Gestalt aufweist.

10. Maske nach Anspruch 1 oder 9, **dadurch gekennzeichnet, dass** der vom Maskenkörper (1) nach oben ragende Haltearm (3) und die zwei gegenüberliegenden, vom Maskenkörper (1) seitlich vorragenden seitlichen Arme (2) mit den Ecken oder Winkelbereichen des dreieckigen Maskenkörpers (1) verbunden sind.

11. Maske nach Anspruch 1, **dadurch gekennzeichnet, dass** ein aus einem starren Polymer bestehendes ringförmiges Element in der Einlassöffnung (4) des Maskenkörpers (1) angeordnet ist.

12. Maske nach Anspruch 11, **dadurch gekennzeichnet, dass** die zwei gegenüberliegenden seitlichen Arme (2) eine allgemein bogenförmige Gestalt aufweisen, wobei die zwei seitlichen Arme (2) zur unteren Rückseite des Maskenkörpers (1) gebogen sind.

13. Maske nach Anspruch 7, **dadurch gekennzeichnet, dass** das Polster (5) und die Membran (8) einen oberen Nasenrückenbereich, einen unteren Bereich und zwei seitliche Bereiche, die den Nasenrückenbereich und den unteren Bereich miteinander verbinden, umfassen, wobei der obere Nasenrückenbereich mit dem Nasenrücken des Patienten in Kontakt ist, der untere Bereich mit der Fläche zwischen der Nase und der Oberlippe des Patienten in Kontakt ist, und die seitlichen gegenüberliegenden Bereiche mit den Flanschbereichen der Nase des Patienten in Kontakt sind, wenn die Maske von einem Patienten getragen wird.

14. Anordnung, umfassend eine Gasabgabevorrichtung und eine Atemmaske nach einem der vorhergehenden Ansprüche.

15. Anordnung nach Anspruch 14, **dadurch gekennzeichnet, dass** die Gasabgabevorrichtung über eine Gasleitung, wie etwa einen flexiblen Schlauch, mit der Atemmaske verbunden ist.

## Revendications

1. Masque respiratoire pédiatrique comprenant :
- un corps (1) de masque comprenant une chambre interne (6) et un orifice d'admission (4) en communication fluidique avec la chambre interne (6),
- un bras de maintien (3) agencé sur le corps (1) de masque et dépassant vers le haut depuis ledit corps (1) de masque,
- un coussin (5) possédant une ouverture (7) conçue pour recevoir au moins une partie du nez du patient, quand le patient porte le masque, et
- deux bras latéraux (2) disposés de chaque côté du corps (1) de masque et dépassant latéralement dudit corps (1) de masque,
et dans lequel le corps (1) de masque, le bras de maintien (3), le coussin (5) et les deux bras latéraux (2) sont solidaires et forment une structure de masque unique en matériau souple résilient,
dans lequel le masque est un masque nasal dimensionné pour s'ajuster sur la région nasale d'un nourrisson, d'un nouveau-né, d'un bébé, d'un tout-petit ou d'un enfant, et dans lequel :
- la structure de masque unique est moulée en une pièce constituée dudit matériau souple résilient,
- le corps de masque a une forme générale triangulaire ou trapézoïdale, et
- l'orifice d'admission est agencé au centre du corps de masque,
**caractérisé en ce que** :
- la structure de masque unique a un poids inférieur à 30 g, et
- le corps de masque a une forme générale triangulaire ou trapézoïdale ayant une première largeur (L1) inférieure à 60 mm et une première hauteur (H1) inférieure à 60 mm.

2. Masque selon la revendication 1, **caractérisé en ce que** le corps de masque a une première largeur (L1) inférieure à 50 mm et une première hauteur (H1) inférieure à 50 mm.

3. Masque selon la revendication 1, **caractérisé en ce que** la structure de masque unique a un poids entre 10 et 20 g.

4. Masque selon l'une quelconque des revendications 1 et 2, **caractérisé en ce que** le corps de masque a une première largeur (L1) entre 40 et 48 mm et une première hauteur (H1) entre 30 et 45 mm.

5. Masque selon l'une quelconque des revendications précédentes, **caractérisé en ce que** le matériau souple résilient formant la pièce de masque unique comprend de la silicone.

6. Masque selon la revendication 1, **caractérisé en ce que** la structure de masque unique a un poids entre 12 et 19 g.

7. Masque selon l'une quelconque des revendications précédentes, **caractérisé en ce que** le coussin (5) comprend au moins une membrane flexible (8), ladite membrane (8) étant constituée dudit matériau souple résilient et formant un seul bloc avec le coussin (5).

8. Masque selon la revendication 1, **caractérisé en ce que** le corps de masque a une épaisseur (T) inférieure à 45 mm.

9. Masque selon la revendication 1, **caractérisé en ce que** le corps (1) de masque a une forme tridimensionnelle généralement triangulaire.

10. Masque selon la revendication 1 ou 9, **caractérisé en ce que** le bras de maintien (3) dépassant vers le haut depuis le corps (1) de masque et les deux bras latéraux opposés (2) dépassant latéralement depuis ledit corps (1) de masque sont reliés aux coins ou régions angulaires du corps (1) de masque de forme triangulaire.

11. Masque selon la revendication 1, **caractérisé en ce qu'**un élément annulaire en polymère rigide est agencé dans l'orifice d'admission (4) du corps (1) de masque.

12. Masque selon la revendication 11, **caractérisé en ce que** les deux bras latéraux opposés (2) ont une forme généralement courbée, lesdits deux bras latéraux (2) étant courbés en direction du côté arrière inférieur du corps (1) de masque.

13. Masque selon la revendication 7, **caractérisé en ce que** le coussin (5) et la membrane (8) comprennent une région de pont nasal supérieure, une région inférieure et deux régions latérales reliant le pont nasal et des régions inférieures, la région de pont nasal supérieure étant en contact avec le pont nasal du patient, la région inférieure étant en contact avec la zone entre le nez et la lèvre supérieure du patient, et les régions opposées latérales étant en contact avec les régions des ailes du nez du patient, lorsque le masque est porté par un patient.

14. Ensemble comprenant un dispositif d'administration de gaz et un masque respiratoire selon l'une quelconque des revendications précédentes.

15. Ensemble selon la revendication 14, **caractérisé en ce que** le dispositif d'administration de gaz est relié au masque respiratoire au moyen d'une conduite de gaz, telle qu'un tuyau souple.
